# EUROPEAN PATENT APPLICATION

(11) **EP 4 740 886 A1**
(43) Date of publication of application: **13.05.2026**
(21) Application number: 24212072.3
(22) Date of filing: 11.11.2024
(51) Int. Cl.: A61B 34/20, A61B 34/10

(54) **GUIDANCE SUPPORT FOR GUIDING A MEDICAL DEVICE**

(71) Applicant: Siemens Healthineers AG, 91301 Forchheim (DE)
(72) Inventor: Fahrig, Rebecca, 91096 Möhrendorf (DE); Kowarschik, Markus, 91052 Erlangen (DE)
(74) Representative: Siemens Healthineers Patent Attorneys

(57) **Abstract**

For guidance support for guiding a medical device (7), image data is received, which represents the medical device (7) inside a hollow organ (8), wherein the medical device (7) extends along a part (9) of the hollow organ (8). A diameter (D) and/or a curvature of the hollow organ (8) at a target position or in a target region (10) for the medical device (7) is determined depending on the image data. Guidance support information for guiding the medical device (7) at least to the target position or the target region (10) is generated depending on the diameter (D) and/or the curvature and depending on at least one geometric property of the medical device (7).

## Description

The present invention is directed to a computer-implemented method for guidance support for guiding a medical device, wherein image data is received, which represents the medical device inside a hollow organ, wherein the medical device extends along a part of the hollow organ. The invention is further directed to a corresponding data processing system for carrying out said computer implemented method, to a medical imaging system comprising said data processing system, and to a corresponding computer program product.

Endovascular procedures, for example catheter-based deployment of a flow-diverting stent, also denoted as flow diverter, to treat a cerebral aneurysm, are often based on pre-interventional imaging using for example computed tomography, CT, cone-beam-CT, CBCT, or magnetic resonance imaging MRI, and/or intra-procedural (real-time) imaging using for example CBCT, fluoroscopy, digital subtraction angiography, DSA, et cetera. The quality of the outcome depends on the intuition, the experience, and the skills of the interventionalist.

Up to now, image information is only interpreted by the interventionalist and then taken into consideration to guide the subsequent clinical decisions and procedural steps. As a consequence difficulties or complications may arise in case it turns out that the used endovascular medical device or a part of it is not suitable for being guided to or through a certain position or region of the vessel structure.

Furthermore, pre-interventional image data may not always be sufficient to select the medical device in an optimal manner since the endovascular intervention itself may change the geometry of the vessel structure. Therefore, similar difficulties or complications may also arise when robotic endovascular devices are used for guiding the medical device.

The same or similar situations may arise in the context of other endoluminar procedures that is applications in hollow organs other than vessel structures, for example in bronchoscopy et cetera.

It is an objective of the present invention to provide an improved concept for image-based guidance support for guiding a medical device inside a hollow organ, which overcomes said drawbacks at least in part.

This objective is achieved by the subject matter of the independent claim. Further implementations and preferred embodiments are subject matter of the dependent claims.

The invention is based on the idea to generate guidance support information for guiding the medical device depending on the diameter of the hollow organ and/or the curvature of the hollow organ at a target position or target region of the hollow organ and depending on at least one geometric property of the medical device.

According to an aspect of the invention, a computer-implemented method for guidance support for guiding a medical device is provided. Therein, image data is received, which represents the medical device inside a hollow organ, wherein the medical device extends along a part of the hollow organ, in particular only along the part of the hollow organ. A diameter of the hollow organ at a target position for the medical device or in a target region for the medical device and/or a curvature of the hollow organ at the target position or in the target region is determined depending on the image data. Guidance support information for guiding the medical device at least to the target position or the target region is generated depending on the diameter of the hollow organ and/or the curvature of the hollow organ and depending on at least one geometric property of the medical device.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for guidance support for guiding a medical device, which is not purely computer-implemented, is obtained by including respective steps of generating the image data, in particular by a medical imaging device.

It is noted that the computer implemented method according to the invention does not comprise any method step for guiding or moving the medical device inside the hollow organ or for inserting the medical device into the hollow organ or another part of the body of a patient.

From each implementation of the computer-implemented method, however, a corresponding method is obtained, which comprises all method steps of the respective implementation of the computer implemented method and a step of guiding the medical device at least to the target position or the target region manually or automatically or semi-automatically.

A hollow organ may for example be understood as an organ that encloses a lumen with its biological tissue. Hollow organs are to be distinguished from parenchymal organs in particular. For example, vessels, in particular blood vessels, bronchi, the panchreatic duct, et cetera are regarded as hollow organs.The medical imaging device may be any device for being inserted into a hollow organ of a patient. The medical device may consist of a single component or may comprise two or more components. A component of the medical device may be intended for being deployed in the hollow organ or, in other words, for staying inside the hollow organ. This may be the case for example for a vessel prosthesis, a vessel implant, a flow diverter, a stent, and so forth. A component of the medical device may, however, also be a tool for deploying another component of the medical device. This may be the case for example for a guide wire or a deployment catheter, et cetera. A component of the medical device may also be another device, which is intended for being removed from the hollow organ after being used. This may be the case for example for a catheter, for example a balloon catheter, et cetera.

Depending on the implementation of the medical imaging device and/or the method being used for generating the image data, the image data can be of different types. For example, it may be an X-ray-based imaging method, such as a monoplanar or biplanar or multiplanar X-ray imaging method, a CBCT method or another suitable method for fluoroscopy or angiography. In case of an X-ray-based imaging method, the image data comprise one or more two-dimensional X-ray images, in particular projection images, and/or a three-dimensional reconstruction and/or one or more slice images obtained from the three-dimensional reconstruction, et cetera. The image data is, in particular, intra-procedural image data.

The image data represents the medical device, in particular, at a current position of the medical device or a specified part of the medical device. According to predetermined interventional planning data or the intention of a user, the medical device or the specified part of the medical device shall be guided from the current position to the target position or the target region or beyond the target position or target region or, in other words, at least to the target position or the target region. The specified part of the medical device may for example be a tip of the medical device or the like. The specified part of the medical device may also be a specified component of the medical device.

The target position or the target region lies, in particular, not within the part of the hollow organ within which the medical device extends according to the image data.

The at least one geometric property of the medical device may therefore comprise at least one geometric property of the whole medical device and/or at least one geometric property of the specified part of the medical device.

The target position or the target region may be obtained by the data processing system for example from the interventional planning data or from user input or from a combination of both.

The at least one geometric property of the medical device may for example be determined based on the image data as well or may be predetermined or specified properties of the medical device.

The diameter of the hollow organ and/or the curvature of the hollow organ may for example be determined by applying a known segmentation algorithm to the image data to obtain a segmented representation or image of the hollow organ. The diameter of the hollow organ and/or the curvature of the hollow organ may then be determined from the segmented representation.

The diameter of the hollow organ in the target region may for example correspond to a minimum diameter or average diameter in the target region. The curvature of the hollow organ in the target region may for example correspond to a maximum curvature or average curvature in the target region.

The image data or data derived from the image data may, for example, be displayed on a display device for assisting the user. This may be a part off the computer implemented method according to the invention which is, however, not necessarily the case.

The guidance support information or a part of it may be provided to the user, for example by being displayed on the display device and/or by being output acoustically, et cetera. The user may then use the guidance support information to guide the medical device at least to the target position or the target region. The user may also decide to terminate the procedure depending on the guidance support information.

The guidance support information or a part of it may also be provided to a endovascular robotic device, which is configured to automatically control a navigation of the medical device in the hollow organ depending on the guidance support information.

In both cases, the result of the endovascular procedure may be improved in terms of quality or in terms of a reduced procedural time by providing the guidance support information as live information or nearly-live information based on the actual conditions of the hollow organ. The risk for damaging the hollow organ by the medical device is reduced.

According to several embodiments, the guidance support information or a part of the guidance support information is output to a user of the medical device and/or the guidance support information or a part of the guidance support information is provided to a control system for automatically controlling a navigation of the medical device, in particular in the hollow organ.

The control system may for example be a control system for controlling the endovascular robotic device depending on the guidance support information.

According to several embodiments, the at least one geometric property of the medical device is determined at least in part depending on the image data. In other words, the at least one geometric property of the medical device is determined depending only on the image data or in part depending on the image data.

Consequently, a modification or variation of the at least one geometric property of the medical device, which is caused due to the insertion of the medical device in the hollow organ is therefore taken into account inherently when determining the at least one geometric property. The at least one geometric property is therefore determined with increased accuracy.

According to several embodiments, the image data comprises at least two projection images, for example X-ray projection images, representing the medical device inside the hollow organ according to at least two respective projection directions.

In particular, each projection image of the at least two projection images represents the medical device inside the hollow organ from a different perspective.

Therefore, the diameter of the hollow organ and/or a curvature of the hollow organ and, in embodiments where at least one geometric property of the medical device is determined at least in part depending on the image data, may be determined with an increased accuracy.

According to several embodiments, the image data comprises a plurality of projection images, for example X-ray projection images, representing the medical device inside the hollow organ according to respective projection directions. A three-dimensional reconstruction representing the medical device inside the hollow organ is generated depending on the plurality of projection images. The diameter of the hollow organ and/or the curvature of the hollow organ is determined depending on the three-dimensional reconstruction.

In particular, each projection image of the at least two projection images represents the medical device inside the hollow organ from a different perspective.

The reconstruction may for example be carried out by applying a known reconstruction technique, for example for CT or CBCT or tomosynthesis.

Therefore, the diameter of the hollow organ and/or a curvature of the hollow organ may be determined with an increased accuracy.

According to several embodiments, the at least one geometric property of the medical device is determined depending on the three-dimensional reconstruction.

Therefore, the at least one geometric property of the medical device is determined at least in part depending on the image data may be determined with an increased accuracy.

According to several embodiments, the at least one geometric property of the medical device comprises a diameter, for example a maximum diameter of the medical device or of a part of the medical device and/or a curvature of the medical device or of the part of the medical device and/or a length of the medical device or of the part of the medical device.

The part of the medical device may be a tip or shaft of the medical device or a component of the medical device as described above.

Consequently, properties, which are particularly relevant for guiding the medical device to or beyond the target position or target region are considered for generating the guidance support information.

The risk for damaging the hollow organ by the medical device or for causing distal vessel occlusions due to emboli is therefore reduced and/or the guidance of the medical device may be simplified or may be carried out more accurately.

According to several embodiments, the guidance support information is generated depending on at least one mechanical property of the medical device.

The at least one mechanical property may for example be predetermined based on a specification of the medical device or a simulation or a model of the medical device.

Thus, the expected behavior, for example a deformation, or friction of the medical device when guiding it to or beyond the target position or target region may be assessed for generating the guidance support information.

The risk for damaging the hollow organ by the medical device is therefore reduced and/or a an unnecessary termination of the procedure may be avoided.

The at least one mechanical property may for example comprise a stiffness of the medical device or of a part of the medical device and/or an elasticity of the medical device or of the part of the medical device and/or a surface lubricity of the medical device or of the part of the medical device.

The at least one mechanical property may for example be determined before carrying out the computer-implemented method according to the invention. In some embodiments, determining the at least one mechanical property, in particular by measuring the medical device, may also be part of the computer-implemented method according to the invention.

The stiffness may, for example, be a tensile stiffness or a shearing stiffness or a bending stiffness or a torsion stiffness. The surface lubricity is, in particular, a lubricity of an outer surface of the medical device. The lubricity is given, for example, by an inverse friction coefficient.

According to several embodiments, the medical device comprises a catheter and/or a vessel prosthesis and/or a vessel implant and/or a flow diverter and/or a stent or and/or guide wire.

According to several embodiments, the guidance support information comprises a risk information concerning a risk of damaging the hollow organ when moving the medical device to the target position or the target region or beyond the target position or the target region. The risk information may for example be provided to the user.

The risk information may for example indicate whether or not a significant risk of damaging the hollow organ exists. The risk information may also comprise a risk value indicating said risk of damaging the hollow organ or a probability of damaging the hollow organ.

The risk for damaging the hollow organ by the medical device is therefore reduced.

According to several embodiments, the guidance support information comprises a recommendation or specification of a navigation maneuver to move the medical device to the target position or the target region or beyond the target position or the target region.

The recommendation or specification of the navigation maneuver may for example be provided to the user or the endovascular robotic device. The user or the endovascular robotic device may then perform the navigation maneuver according to the recommendation or specification.

The recommendation or specification of the navigation maneuver may for example be a recommendation or specification to move the medical device in a certain manner and/or to change an orientation or state of the medical device in a certain manner when guiding the medical device to the target position or the target region or beyond.

The risk for damaging the hollow organ by the medical device is therefore reduced and/or the guidance of the medical device may be simplified or may be carried out more accurately.

According to several embodiments, the guidance support information comprises a recommendation not to move the medical device to the target position or the target region or beyond the target position or the target region.

The recommendation or specification may for example be provided to the user or the endovascular robotic device. The user may then decide to terminate the procedure or replace the medical device or a component of it by a more suitable alternative based on the recommendation. The endovascular robotic device may decide to terminate the procedure based on the recommendation. The risk for damaging the hollow organ by the medical device is therefore reduced.

According to several embodiments, the guidance support information comprises the diameter of the hollow organ and/or the curvature of the hollow organ.

The diameter of the hollow organ and/or the curvature of the hollow organ may then for example be output to the user. The risk for damaging the hollow organ by the medical device is therefore reduced and/or the guidance of the medical device may be simplified or may be carried out more accurately.

The risk information, the recommendation or specification of the navigation maneuver and/or the recommendation not to move the medical device may for example be generated depending on a comparison of the diameter of the hollow organ and/or the curvature of the hollow organ with the at least one geometric property of the medical device, for example the diameter or maximum diameter of the medical device or of the part of the medical device, the curvature of the medical device or of the part of the medical device and/or the length of the medical device or of the part of the medical device. They may, alternatively or in addition, be generated depending on the at least one mechanical property of the medical device.

According to several embodiments, a blood flow in the hollow organ is inferred based on the image data. The guidance support information includes the inferred blood flow.

The blood flow may for example be inferred by analyzing the image data with respect to regions of higher or lower blood flow across the hollow organ's lumen and/or lumen-blocking structures such as calcifications or other blockages.

According to a further aspect of the invention, a data processing system that is configured to carry out a computer-implemented method according to the invention is provided.

In the present disclosure, the terms "data processing system" and "at least one data processing device" can be used interchangeably. In particular, a data processing device can be understood to mean a data processing device that contains a processing circuit. The data processing device can therefore, in particular, process data for the purpose of performing computing operations. This may also include operations for performing indexed access to a data structure, for example a look-up table, LUT, as well as a data processing method implemented in hardware.

The data processing device may include, in particular, one or more computers, one or more microcontrollers and/or one or more integrated circuits, for example one or more application-specific integrated circuits, ASIC, one or more field-programmable gate arrays, FPGA, and/or one or more systems-on-a-chip, SoC. The data processing device may also include one or more processors, for example one or more microprocessors, one or more central processing units, CPUs, one or more graphics processing units, GPUs, and/or one or more signal processors, in particular one or more digital signal processors, DSPs. The data processing device may also include a physical or virtual network of computers or other of the mentioned units.

In various embodiments, the data processing device includes one or more hardware and/or software interfaces and/or one or more storage units.

A storage unit may be a volatile data memory, for example a dynamic random access memory, DRAM, or a static random access memory, SRAM, or a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a medical imaging system is provided. The medical imaging system comprises a data processing system according to the invention and a medical imaging device, which is configured to generate the image data.

According to several embodiments, the medical imaging device is implemented as an X-ray based imaging device, for example a fluoroscopy device and/or a C-arm X-ray device or a biplanar or multiplanar X-ray device.

According to several embodiments, the medical imaging system comprises an endovascular robotic device, which is configured to automatically control a navigation of the medical device, in particular in the hollow organ, depending on the guidance support information.

Further embodiments of the medical imaging system according to the invention follow directly from the various embodiments of the computer-implemented method according to the invention and vice versa. In particular, individual features and corresponding explanations as well as advantages relating to the various implementations of the computer-implemented method according to the invention can be transferred analogously to corresponding implementations of the medical imaging system according to the invention. In particular, the medical imaging system according to the invention is designed or programmed to carry out the computer-implemented method according to the invention. In particular, the medical imaging system according to the invention carries out the computer-implemented method according to the invention.

According to a further aspect of the invention, a computer program comprising instructions is provided. When the instructions are executed by a data processing system, the instructions cause the data processing system to carry out a computer-implemented method according to the invention.

The instructions may be provided as program code, for example. The program code can for example be provided as binary code or assembler and/or as source code of a programming language, for example C, and/or as program script, for example Python.

According to a further aspect of the invention, a computer-readable storage medium, in particular a tangible and/or non-transient computer readable storage medium, storing a computer program according to the invention is provided.

The computer program and the computer-readable storage medium are respective computer program products comprising the instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows schematically an exemplary embodiment of a medical imaging system according to the invention;
- FIG 2: shows a schematic flow diagram of an exemplary embodiment of a computer implemented method for guidance support for guiding a medical device according to the invention; and
- FIG 3: shows schematically a hollow organ and a medical device inside the hollow organ.

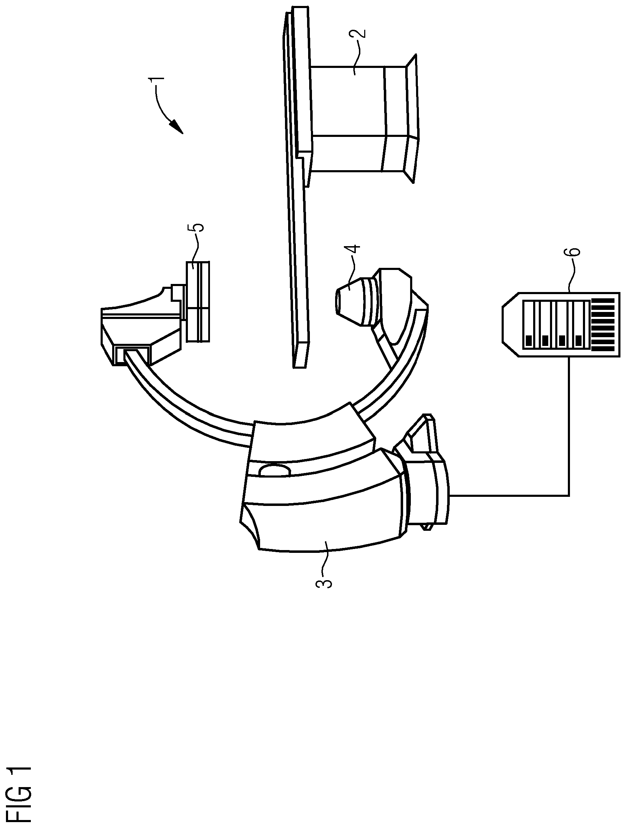

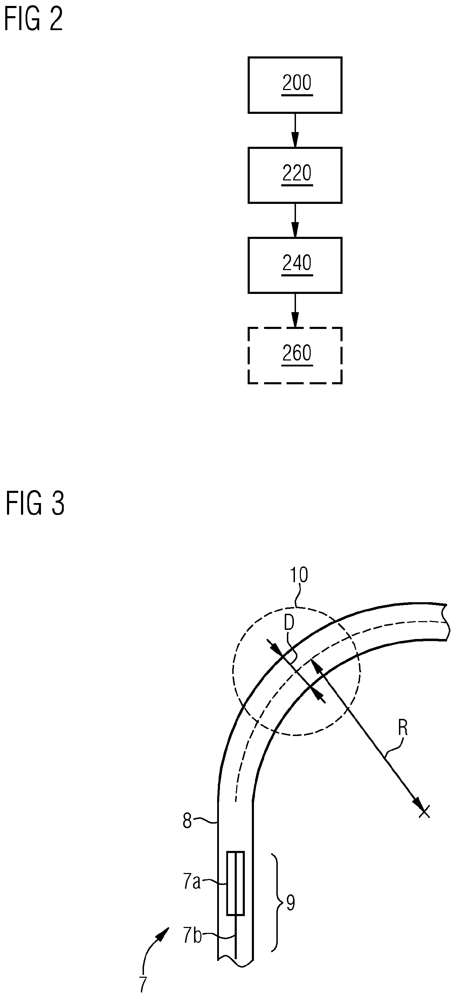

FIG 1 shows schematically an exemplary embodiment of a medical imaging system 1 according to the invention. The medical imaging system 1 comprises a medical imaging device 3 that is set up to generate image data, which represents a medical device 7 inside a hollow organ 8 of a patient, wherein the medical device 7 extends along a part 9 of the hollow organ 8, as shown schematically in FIG 3. In the example, the hollow organ 8 is for example a vessel structure.

The imaging device 3 is shown as an example of a C-arm X-ray device with an X-ray source 4 and an X-ray detector 5. The imaging device 3 may therefore be designed, for example, as a biplanar or multiplanar fluoroscopy device. However, the following explanations can be applied analogously to other imaging devices 3. The patient may be placed on a patient table 2 of the medical imaging system 1.

The imaging arrangement 1 comprises a data processing system 6 according to the invention, which is configured to carry out a computer-implemented method for guidance support for guiding the medical device 7 based on the image data.

FIG 2 shows a schematic block diagram of an exemplary embodiment of such a computer-implemented method according to the invention.

In step 200, the image data is received and in step 220, diameter D and/or a curvature of the hollow organ 8 at a target position or in a target region 10 for the medical device 7 is determined depending on the image data. The curvature of the hollow organ corresponds to the inverse of a respective radius of curvature R. In step 240, guidance support information for guiding the medical device 7 at least to the target position or the target region 10 is generated depending on the diameter of the hollow organ D and/or the curvature of the hollow organ 1/R and depending on at least one geometric property of the medical device 7.

In optional step 260, the guidance support information or a part of the guidance support information is output to a user of the medical device 7 or the guidance support information or a part of the guidance support information is provided to a control system for automatically controlling a navigation of the medical device 7, for example to a control system of an endovascular robotiv device, which is configured to automatically control the navigation of the medical device 7 depending on the guidance support information.

In the example of FIG 3, the medical device 7 comprises two components 7a, 7b. A first component 7a may for example be a catheter or a flow diverter or a flow diverter encapsulated inside a catheter or another device to be deployed inside the hollow organ 8 or another organ of the patient. A second component 7b may for example be a guide wire for navigating the medical device 7 or the first component 7a, respectively.

By means of the invention, the drawbacks of conventional methods may be overcome at least in part. Various further embodiments and extensions of the described embodiments are possible.

In some embodiments, quantitative data based on the intra-procedural image data, for example in combination with pre-procedural image data, are generated and provided to the interventionalist or to a clinical decision support system in order to guide subsequent workflow steps of an endovascular procedure, enhance accuracy and safety, and thus finally enhance patient outcome.

For example, real-time information may be provided.

In some embodiments, the real-time information comprises distances to predefined, for example user-specified, landmarks, such as proximal and/or distal landing zones of a stent, or anatomical landmarks, such as vessel bifurcations, aneurysm necks, et cetera.

In some embodiments, these distances may be measured in 3D along the respective vessel's centerline. To this end, the centerline may be determined in a current 3D angiographic image. In addition, a device tip may be detected and/or the device tip may be backprojected into the 3D angiographic image.

In some embodiments, the real-time information comprises vessel diameter estimates at the device tip. This can for example be based on intra-procedural angiographic 3D imaging or on vessel diameter estimates derived from 2D angiographic images from at least one viewing direction. If an angiographic biplane system is used, there are two viewing directions available. If, however, more than two viewing directions shall be used to improve the accuracy of the vessel diameter estimate, the plane/planes may be re-angulated.

In some embodiments, the real-time information comprises geometric measures to estimate in how far the patient's vasculature has been deformed due to the insertion of the medical device devices in comparison to the patient's vasculature prior to the intervention, which may be derived from a pre-interventional angiographic image.

In some embodiments, the displacement of centerline points may be continously estimated, for example by estimating distances of corresponding centerline points in 3D. Also the changes of the curvatures may be continously estimated. These estimates may be derived approximately in real-time from 2D images, for example using a biplane angiographic imaging system such that two viewing directions are available for each time point.

In some embodiments, a continuous tomosynthesis, that is a limited-angle 3D imaging method, may be used to accurately depict geometric deviations of from a reference 3D dataset.

In some embodiments, 3D images are continuously acquired using tomosynthesis and the corresponding results are compared to a reference 3D image in order to update geometric parameters and metrics as provided in real-time, for example a vessel-to-device diameter ratio, a vessel movement, a device tip location, et cetera.

In some embodiments, an analysis of flow dynamics is carried out, for example bolus arrival times at representative anatomical landmarks or downstream parenchymal blushes may be estimated. The results may be compared to corresponding reference data that has been acquired before.

In some embodiments, modifications in X-ray attenuation over the cardiac cycle in parenchyma, for example lung parenchyma, may be analysed. For example, recanalizing vessels, for example in the lung, may lead to a change in X-ray attenuation over the patient's cardiac cycle because the amounts of blood during the cardiac phases vary. This effect can be exploited to generate metrics, for example based on estimates of X-ray attenuation from real-time 2D images, that assess the success of the recanalization.

Alternatively or in addition to the real-time information, intra-procedural but not real-time information may be provided.

In some embodiments, an assessment of aneurysm coils protruding into the parent artery may be made. This unfortunate situation can be assessed by analyzing real-time image data under the assumption that a proper viewing direction is selected that allows for a perpendicular view onto the parent artery and the aneurysm. Based on 3D image information, such a viewing direction can be selected and automatically.

In some embodiments, an assessment of how well the neck of a saccular aneurysm is covered as soon as a first coil is placed into the aneurysm dome may be made. To this end, a high-resolution, in particular non-contrast-enhanced, 3D angiography image may be used and a proper view of the aneurysm neck may be analyzed. The result can be provided to the interventionalist as a 3D rendering of the aneurysm neck using a perpendicular viewing direction.

In some embodiments, a visual display of the respective parameters is provided. This may be done by displaying the respective numbers or using more intuitive representations or visualizations, for example traffic lights, color bars, progress bars, et cetera.

In some embodiments, acoustic signals may be output when the medical device is approaching a particular landmark or moving away from it.

In some embodiments, the assessment of the aforementioned real-time parameters, such as distances to landmarks, diameters, displacements and curvature changes, are performed periodically. Alternatively or in addition, the assessment of the aforementioned real-time parameters are adapted to particular conditions, for example a speed of the device tip (higher speed imply a more frequent parameter assessment), or a distance to particular landmarks (the closer the device gets to a particular landmark, the more frequently the parameters may be assessed).

In some embodiments, intravascular imaging, for example optical coherence tomography, OCT, or intravascular Ultrasound, IVUS, are additionally used to provide further real-time data to guide endovascular therapies.

Further advantages of several embodiments include a more objective assessment of endovascular therapy delivery, an enabling of a more precise deployment of endovascular devices, for example stents, avoiding vasospasm that may be caused by inserting large-bore catheters into a relatively narrow vessel.

Further advantages of several embodiments include the possibility to standardize endovascular procedures and to enhance patient outcomes, prevention of using and/or deployment of inappropriate endovascular devices, for example stents with a diameter that is too large for the target vessel, stents that are too long for the target location in the target vessel, or guidelines or catheters that are too stiff for the patient's vasculature.

Independent of the grammatical term usage, individuals with male, female or other gender identities are included within the term.

## Claims

1. Computer-implemented method for guidance support for guiding a medical device (7), wherein
- image data is received, which represents the medical device (7) inside a hollow organ (8), wherein the medical device (7) extends along a part (9) of the hollow organ (8);
- a diameter (D) and/or a curvature of the hollow organ (8) at a target position or in a target region (10) for the medical device (7) is determined depending on the image data; and
- guidance support information for guiding the medical device (7) at least to the target position or the target region (10) is generated depending on the diameter (D) and/or the curvature and depending on at least one geometric property of the medical device (7).

2. Computer-implemented method according to claim 1, wherein the at least one geometric property of the medical device (7) is determined at least in part depending on the image data.

3. Computer-implemented method according to one of the preceding claims, wherein the image data comprises at least two projection images representing the medical device (7) inside the hollow organ (8) according to at least two respective projection directions.

4. Computer-implemented method according to one of claims 1 or 2, wherein
- the image data comprises a plurality of projection images representing the medical device (7) inside the hollow organ (8) according to respective projection directions;
- a three-dimensional reconstruction is generated depending on the plurality of projection images; and
- the diameter (D) and/or the curvature is determined depending on the three-dimensional reconstruction.

5. Computer-implemented method according to one of the preceding claims, wherein the at least one geometric property of the medical device (7) comprises a diameter of the medical device (7) or of a part (7a, 7b) of the medical device (7) and/or a curvature of the medical device (7) or of the part (7a, 7b) of the medical device (7) and/or a length of the medical device (7) or of the part (7a, 7b) of the medical device (7) and/or a ratio of an inner diameter to an outer diameter of the medical device (7) or of the part (7a, 7b) of the medical device (7) .

6. Computer-implemented method according to one of the preceding claims, wherein the guidance support information is generated depending on at least one mechanical property of the medical device (7).

7. Computer-implemented method according to claim 6, wherein the at least one mechanical property comprises a stiffness of the medical device (7) or of a part (7a, 7b) of the medical device (7) and/or an elasticity of the medical device (7) or of the part (7a, 7b) of the medical device (7) and/or a surface lubricity of the medical device (7) or of the part (7a, 7b) of the medical device (7).

8. Computer-implemented method according to one of the preceding claims, wherein the guidance support information or a part of the guidance support information is output to a user of the medical device (7).

9. Computer-implemented method according to one of the preceding claims, wherein the guidance support information or a part of the guidance support information is provided to a control system for automatically controlling a navigation of the medical device (7).

10. Computer-implemented method according to one of the preceding claims, wherein the medical device (7) comprises a catheter and/or a vessel prosthesis and/or a vessel implant and/or a flow diverter and/or a stent and/or a guide wire.

11. Computer-implemented method according to one of the preceding claims, wherein the guidance support information comprises
- a risk information concerning a risk of damaging the hollow organ (8) when moving the medical device (7) to the target position or the target region (10) or beyond the target position or the target region (10); and/or
- a recommendation or specification of a navigation maneuver to move the medical device (7) to the target position or the target region (10) or beyond the target position or the target region (10); and/or
- a recommendation not to move the medical device (7) to the target position or the target region (10) or beyond the target position or the target region (10).

12. Data processing system (6), which is configured to carry out a computer-implemented method according to one of the preceding claims.

13. Medical imaging system (1) comprising a data processing system (6) according to claim 12 and a medical imaging device (3), which is configured to generate the image data.

14. Medical imaging system (1) according to claim 13, further comprising an endovascular robotic device, which is configured to automatically control a navigation of the medical device (7) depending on the guidance support information.

15. Computer program product comprising instructions, which, when executed by a data processing system (6), causes the data processing system (6) to carry out a computer-implemented method according to one of claims 1 to 11.
